(19) Europäisches Patentamt
European Patent Office
Office européen des brevets

(11) Veröffentlichungsnummer: **0 415 278 A1**

(12) **EUROPÄISCHE PATENTANMELDUNG**

(21) Anmeldenummer: **90116216.4**

(22) Anmeldetag: **24.08.90**

(51) Int. Cl.5: **C07C 211/52**, C07C 245/08, G02F 1/35, C08G 77/388

(30) Priorität: **01.09.89 DE 3929053**

(43) Veröffentlichungstag der Anmeldung: **06.03.91 Patentblatt 91/10**

(84) Benannte Vertragsstaaten: **BE DE FR GB IT NL**

(71) Anmelder: **BASF Aktiengesellschaft**
**Carl-Bosch-Strasse 38**
**W-6700 Ludwigshafen(DE)**

(72) Erfinder: **Brox, Wolfgang, Dr.**
**Im Grund 13**
**W-6900 Heidelberg(DE)**
Erfinder: **Funhoff, Dirk, Dr.**
**Panoramastrasse 3 a**
**W-6900 Heidelberg(DE)**
Erfinder: **Licht, Ulrike, Dr.**
**Meerfeldstrasse 62**
**W-6800 Mannheim 1(DE)**
Erfinder: **Fuchs, Harald, Dr.**
**Boehlweg 32**
**W-6719 Carlsberg(DE)**
Erfinder: **Schrepp, Wolfgang, Dr.**
**Sitzbuchweg 114**
**W-6900 Heidelberg(DE)**

# (54) Endständig ethylenisch ungesättigte Verbindungen sowie ihre Verwendung in der nichtlinearen Optik.

(57) Die neuen Verbindungen der allgemeinen Formel I mit nichtlinear optischen Eigenschaften,

$$CH_2=CH-X-\overset{R}{N}-Y \qquad (I)$$

worin die Variablen die folgende Bedeutung haben:
X geradkettiger oder verzweigter Alkandiylrest mit 1 bis 20 C-Atomen oder
X geradkettiger oder verzweigter Alkandiylrest mit 2 bis 20 C-Atomen, dessen Kohlenstoffkette durch -O-, -S- und/oder -NR- unterbrochen ist;
R Wasserstoffatom, geradkettiger oder verzweigter Alkylrest mit 1 bis 6 C-Atomen oder Cycloalkylrest,
Y nicht-zentrosymmetrischer, ein leicht polarisierbares konjugiertes $\pi$-Elektronensystem sowie mindestens eine endständige Elektronenakzeptorgruppe enthaltender Rest;
sind hervorragend als nichtlinear optische Materialien für nichtlinear optische Anordnungen sowie für die Herstellung von Polymeren mit nichtlinear optischen Eigenschaften geeignet. Insbesondere dienen sie der Herstellung von Organopolysiloxanen mit nichtlinear optischen Eigenschaften, welche Seitengruppen der allgemeinen Formel Ia enthalten,

EP 0 415 278 A1

Xerox Copy Centre

$$-CH_2-CH_2-X-\overset{R}{\overset{|}{N}}-Y \qquad \text{(Ia)}$$

worin die Variablen die vorstehend angegebene Bedeutung haben. Diese neuartigen Organopolysiloxane mit nichtlinear optischen Eigenschaften sind wiederum hervorragend für die Herstellung von Langmuir-Blodgett-Schichten geeignet.

# ENDSTÄNDIG ETHYLENISCH UNGESÄTTIGTE VERBINDUNGEN SOWIE IHRE VERWENDUNG IN DER NICHTLINEAREN OPTIK

Die vorliegende Erfindung betrifft die neuen Verbindungen der allgemeinen Formel I mit nichtlinear optischen Eigenschaften,

$$CH_2{=}CH{-}X{-}\overset{R}{\overset{|}{N}}{-}Y \qquad (I)$$

worin die Variablen die folgende Bedeutung haben:
X geradkettiger oder verzweigter Alkandiylrest mit 1 bis 20 C-Atomen oder
X geradkettiger oder verzweigter Alkandiylrest mit 2 bis 20 C-Atomen, dessen Kohlenstoffkette durch -O-, -S- und/oder -NR- unterbrochen ist;
R Wasserstoffatom, geradkettiger oder verzweigter Alkylrest mit 1 bis 6 C-Atomen oder Cycloalkylrest,
Y nicht-zentrosymmetrischer, ein leicht polarisierbares konjugiertes $\pi$-Elektronensystem sowie mindestens eine endständige Elektronenakzeptorgruppe enthaltender Rest.

Außerdem betrifft die Erfindung die Verwendung der neuen Verbindungen I in der nichtlinearen Optik sowie für die Herstellung von neuen Polymeren mit nichtlinear optischen Eigenschaften.

Die nichtlineare Optik beschäftigt sich ganz allgemein mit der Wechselwirkung elektromagnetischer Felder in unterschiedlichen Stoffen und dem damit verbundenen feldabhängigen Brechungsindex in diesen Stoffen.

Ganz allgemein emittiert ein Stoff Licht, wenn in ihm Dipole schwingen, wobei die Frequenz der emittierten Lichtwelle gleich der Schwingungsfrequenz der Dipole ist. Enthalten die schwingenden Dipole mehrere Frequenzkomponenten, kommen diese alle in dem vom betreffenden Stoff emittierten Licht vor. Sofern die räumliche Ausdehnung des Stoffs größer als die Wellenlänge des emittierten Lichts ist, sollten die im Stoff schwingenden identischen Dipole möglichst in gleicher Richtung und mit einer Phasendifferenz schwingen, welche dafür sorgt, daß das von einem Volumenelement emittierte Licht nicht durch destruktive Interferenz mit dem von einem anderen Volumenelement emittierten Licht wieder gelöscht wird.

In einem polarisierbaren Stoff wird durch ein von außen angelegtes elektrisches Feld

$\vec{E}$ eine

makroskopische Polarisation

$\vec{P}$ hervorgerufen,

welche als Dipolmoment pro Volumen definiert ist.

Enthält der polarisierbare Stoff keine permanenten molekularen Dipole, resultiert das Dipolmoment und damit die makroskopische Polarisation

$\vec{P}$

aus der Verschiebung der Elektronen um den Betrag

$\vec{d}$ aus

ihrer Ruhelage, d.h. dem Schwerpunkt der positiven Ladung, heraus. Enthält der polarisierbare Stoff dagegen permanente Dipole, ändert sich durch das angelegte elektrische Feld

$\vec{E}$ das

permanente Dipolmoment nach dem selben Mechanismus.

Solange die Verschiebung

$$\vec{d}$$

proportional zum elektrischen Feld

$$\vec{E}$$

bleibt, ist auch die Polarisation

$$\vec{P}$$

proportional zum elektrischen Feld

$$\vec{E},$$

was in der bekannten linearen Gleichung 1

$$\vec{P} = \varepsilon_0 \chi \vec{E} \qquad \text{Gl. 1}$$

zum Ausdruck kommt. In der Gleichung 1 bezeichnet $\varepsilon_0$ die absolute Dielektrizitätskonstante und $\chi$ die dielektrische Suszeptibilität.

Wird das von außen angelegte elektrische Feld

$$\vec{E}$$

verstärkt, muß naturgemäß jeder Stoff oberhalb einer für ihn spezifischen Feldstärke eine Abweichung von linearen Gesetz gemäß der Gleichung 1 zeigen. Das mechanische Analogon hierzu ist die Abweichung vom Hookschen Gesetz bei Überbelastung einer Feder. Solche Abweichungen von der Linearität werden mathematisch am einfachsten durch Hinzufügen einer Parabel und höherer Potenzen der Variablen behandelt, d.h., man entwickelt die nichtlineare Funktion nach Potenzen der Variablen

$$\vec{E},$$

woraus Gleichung 2,

$$\vec{P} = \varepsilon_0 (\chi^{(1)} \vec{E} + \chi^{(2)} \vec{E}\vec{E} + \chi^{(3)} \vec{E}\vec{E}\vec{E} \; ....) \qquad \text{Gl. 2}$$

die grundlegende Gleichung der nichtlinearen Optik, resultiert. Hierin bedeutet

- $\chi^{(1)}$ die dielektrische Suszeptibilität erster Ordnung, welche letztlich für lineares optisches Verhalten des betreffenden Stoffs verantwortlich ist,
- $\chi^{(2)}$ die dielektrische Suszeptibilität zweiter Ordnung, welche das nichtlineare optische Verhalten zweiter Ordnung des betreffenden Stoffs hervorruft, und
- $\chi^{(3)}$ die dielektrische Suszeptibilität dritter Ordnung, auf welche das nichtlineare optische Verhalten dritter Ordnung des betreffenden Stoffs zurückgeht.

Sowohl $\chi^{(2)}$ als auch $\chi^{(3)}$ sind Materialkonstanten, welche von der Molekülstruktur, von der Kristallstruktur, von der Frequenz des Lichts und im allgemeinen auch von der Temperatur abhängig sind. Sie können bekanntermaßen mit Hilfe der dynamisch holographischen Methode der "Vier-Wellen-Mischung" ermittelt werden, wie sie von

- W.W. Schkunow et al. in Spektrum der Wissenschaft, Februar 1986, Seiten 92 bis 97; und
- J.P. Huignard et al. in SPIE Band 215, Recent Advances in Holography, Seiten 178 bis 182, 1980,

beschrieben wird.

Stoffe mit einer feldstärkenabhängigen dielektrischen Suszeptibilität $\chi^{(2)}$, d.h. mit nichtlinear optischen

Eigenschaften zweiter Ordnung, haben eine Reihe dispersiver Prozesse, wie
- die Frequenzverdoppelung (second harmonic generation, SHG), welche die Erzeugung von Licht der verglichen mit dem eingestrahlten Licht halben Wellenlänge erlaubt;
- den elektrooptischen Effekt (Pockels-Effekt), welcher eine Änderung des Brechungsindex bei angelegtem elektrischen Feld ermöglicht, oder
- die Summen- und Differenzfrequenzmischung sowie die Frequenzmischung, welche die kontinuierliche Abstimmung von Laserlicht gestattet,

zur Folge, woraus eine Vielzahl technischer Anwendungen resultieren. Beispielhaft seien die elektrooptischen Schalter, die Frequenz- und Intensitätssteuerung in der Lasertechnik, die Holographie, die Informationsverarbeitung und die integrierte Optik genannt.

Stoffe mit einer feldstärkenabhängigen dielektrischen Suszeptibilität $\chi^{(3)}$, d.h. mit nichtlinear optischen Eigenschaften dritter Ordnung, eignen sich u.a. zur Herstellung rein-optischer Schalter und damit als Wellenleiter zur Konstruktion rein-optischer Computer.

Weitere Anwendungsmöglichkeiten werden in der Publikation von
- D.R. Ulrich, "Nonlinear Optical Polymer Systems and Devices", in Molecular Crystals und Liquid Crystals, Band 180, Seiten 1 bis 31, 1988,

beschrieben. Aus diesem Artikel geht außerdem die wachsende Bedeutung von Polymeren mit nichtlinear optischen Eigenschaften hervor, von denen man sich erhofft, daß sie sich durch
- Ansprechzeiten unterhalb einer Picosekunde,
- hohe, nichtresonante Nichtlinearitäten,
- bei Gleichstrom niedrigen dielektrischen Konstanten,
- niedrigen Schaltenergien,
- einen breiten Frequenzbereich,
- eine geringe Absorption,
- die Abwesenheit von Diffusionsproblemen,
- die Möglichkeit der Resonanzverstärkung,
- ihre einfache Herstellbarkeit und Verarbeitbarkeit sowie die Möglichkeit, sie in einfacher Weise zu modifizieren,
- ihre Handhabbarkeit und Verwendbarkeit bei Raumtemperatur,
- ihre Stabilität gegenüber Umwelteinflüssen und
- ihre mechanische und strukturelle Stabilität

auszeichnen und daher zunehmend die seit langem bekannten anorganischen und organischen kristallinen Stoffe mit nichtlinear optischen Eigenschaften verdrängen werden.

Bekanntermaßen haben die in dem Artikel von D.R. Ulrich aufgeführten Polymere wie alle Stoffe linear optische und nichtlinear optische Eigenschaften dritter Ordnung, wogegen die nichtlinear optischen Eigenschaften zweiter Ordnung an das Vorhandensein einer nicht-zentrosymmetrischen Molekülstruktur und/oder einer nicht-zentrosymmetrischen Molekülanordnung im Kristall gebunden sind. Außerdem muß ein Polymer eine dielektrische Suszeptibilität $\chi^{(2)}$ von mindestens $10^{-8}$, vorzugsweise $10^{-7}$ esu, aufweisen, um für die vorstehend genannten Verwendungszwecke geeignet zu sein, was hohe Anforderungen an die Molekülstruktur der Polymeren als solche, ihre Herstellbarkeit und an die einheitliche räumliche Ausrichtbarkeit der darin enthaltenen Gruppen mit nichtlinear optischen Eigenschaften zweiter Ordnung stellt. Erst wenn diese Anforderungen erfüllt sind, können die sonstigen Vorteile, welche den Polymeren zu eigen sind oder welche man sich von ihnen erhofft, nutzbringend angewandt oder realisiert werden.

Geordnete monomolekulare Schichten sind gleichfalls bekannt. Sie werden von Verbindungen gebildet, welche ein polares und daher hydrophiles Molekülende und einen unpolaren und daher hydrophoben langkettigen Rest aufweisen. Verbindungen dieser Art werden allgemein auch als Amphiphile bezeichnet. Zur Schichtbildung werden die Amphiphile auf eine Wasseroberfläche aufgebracht, auf welcher sie sich ausbreiten, wobei ihr polares Ende in die wäßrige Phase eintaucht und ihr hydrophober langkettiger Rest aus der wäßrigen Phase herausragt. Werden die Verbindungen dann auf der Wasseroberfläche mittels einer Barriere zusammengeschoben, ordnen sie sich ab einem bestimmten Oberflächendruck zu einer geordneten monomolekularen Schicht, in welcher die hydrophoben langkettigen Reste einheitlich räumlich ausgerichtet sind. Die Umwandlung in eine solche geordnete monomolekulare Schicht macht sich im Druck-Flächen-Diagramm, welches während des Zusammenschiebens der betreffenden Verbindung aufgenommen wird, durch einen kräftigen Druckanstieg bemerkbar. Dieser Druckanstieg resultiert aus dem erhöhten Widerstand, den die nun geordnete monomolekulare Schicht einer weiteren Kompression durch die Barriere entgegensetzt.

Die so hergestellte geordnete monomolekulare Schicht kann in einfacher Weise auf die Oberfläche von Trägern aufgezogen werden. Dies geschieht üblicherweise durch Eintauchen der Träger in die wäßrige

Phase und Wiederherausziehen, wodurch die geordnete monomolekulare Schicht von der Wasseroberfläche auf die Trägeroberfläche übertragen wird, wobei z.B. bei hydrophoben Oberflächen wie reinen Siliciumflächen die unpolaren Molekülenden der Verbindungen an der Trägeroberfläche haften. Ist diese Übertragung vollständig, spricht man allgemein von einem Übertragungsverhältnis von 1.

Auf die Oberfläche der auf dem Träger befindlichen geordneten monomolekularen Schicht kann mindestens eine weitere Schicht dieser Art aufgezogen werden. Üblicherweise wird dabei diese weitere geordnete monomolekulare Schicht auf der ersten so aufgetragen, daß die polaren Molekülenden der Verbindungen beider Schichten aneinander zugekehrt sind. Diese räumliche Anordnung wird auch als "Kopf-Kopf-Schwanz-Schwanz"-Orientierung oder als "Y-deposition" bezeichnet. Bringt man auf diese Doppelschicht eine dritte Schicht auf, ordnet sie sich in entsprechender Weise so an, daß ihre hydrophoben langkettigen Reste den betreffenden Resten der zweiten Schicht zugewandt sind, wogegen ihre polaren Molekülenden nach außen weisen.

Haben die Verbindungen, aus welchen diese geordneten monomolekularen Multischichten bestehen, ein permanentes Dipolmoment, dann resultiert aufgrund der "Y-deposition" nur bei einer ungeraden Anzahl von übereinanderliegenden Schichten eine makroskopische Polarisation P, welche auch nur auf die oberste der Schichten zurückgeht. Bei einer geraden Anzahl resultiert naturgemäß eine makroskopische Polarisation P von Null, weil sich die Dipolmomente der einzelnen Schichten aufgrund ihrer gegenseitigen Ausrichtung aufheben.

Will man dagegen bei einer in "Y-deposition" aufgetragenen geordneten monomolekularen Multischicht die maximal mögliche makroskopische Polarisation P zumindest angenähert erzielen, müssen zwischen die einzelnen Schichten aus Verbindungen mit einem permanenten Dipol geordnete monomolekulare Schichten aus Verbindungen ohne Dipolmoment in "Y-deposition" eingelagert werden, wodurch eine alternierende Schichtfolge resultiert, in der alle vorhandenen permanenten Dipole einheitlich ausgerichtet sind.

Bekanntermaßen werden sowohl die geordneten monomolekularen Monoschichten als auch die entsprechenden Multischichten als "Langmuir-Blodgett-Schichten" bezeichnet. Das Verfahren zu ihrer Herstellung sowie die hierzu verwendeten Apparaturen werden üblicherweise unter dem stehenden Fachbegriff "Langmuir-Blodgett-Technik" zusammengefaßt. Der Kürze halber werden im folgenden nur noch diese Fachbegriffe verwendet.

Verbindungen mit nichtlinear optischen Eigenschaften sind beispielsweise aus der GB-A-2 204 053 bekannt. Hierbei handelt es sich um Azoverbindungen, welche in üblicher und bekannter Weise durch die Azokupplung von 4-Tetradecylanilin und Naphth-2-ol, Naphth-1-ol oder 1-Naphthylamin, von 4-Nitroanilin und 1-Naphthylamin oder 1-(N-Tetradecyl)-naphthylamin, von 4-Aminobenzoesäure und 1-(N-Tetradecyl)-naphthylamin, von 4-Aminobenzonitril und 1-(N-Tetradecyl)-naphthylamin oder 1-(N-Heptyl)-naphthylamin, von 4-Aminobenzoesäure und N-Decyl-N-methyl-m-toluidin und von 4-Aminobenzonitril und N-Tetradecylanilin herstellen lassen. Zwar lassen sich diese bekannten Verbindungen in der nichtlinearen Optik und für die Herstellung von Langmuir-Blodgett-Schichten verwenden, indes weisen sie keine endständig ethylenisch ungesättigte Gruppe auf, so daß sie nicht für die Herstellung von Polymeren mit nichtlinear optischen Eigenschaften geeignet sind.

Verbindungen wie 4-(9-Decenthia)-4′-cyano-stilben oder 4-(4-Pentenoxy)-4′-nitro-biphenyl, welche sowohl nichtlinear optische Eigenschaften aufweisen als auch für die Herstellung von Polymeren, insbesondere von Organopolysiloxanen, mit nichtlinear optischen Eigenschaften geeignet sind, sind aus der US-A-4 762 912 bekannt. Außerdem geht hieraus ein Organopolysiloxan hervor, welches 5-(4′-Nitro-biphenyl-4-oxy)-pent-1-yl-Reste als Seitengruppen mit nichtlinear optischen Eigenschaften enthält. Weitere Organopolysiloxane dieser Art, welche 4-(4′-Cyano-biphenyl-4-oxy)-but-1-yl-, 5-(4′-Cyano-biphenyl-4-oxy)-pent-1-yl- oder 6-(4′-Cyano-biphenyl-4-oxy)-hex-1-yl-Reste enthalten, sind aus der EP-A-0 141 512 bekannt. Indes wird hierin nicht angegeben, ob diese Seitengruppen der Organopolysiloxane nichtlinear optische Eigenschaften aufweisen.

Sowohl in der US-A-4 762 912 als auch in der EP-A-0 141 512 erfolgt die zur Ausnutzung der nichtlinear optischen Eigenschaften zweiter Ordnung notwendige einheitliche räumliche Ausrichtung der Seitengruppen der Organopolysiloxane über das Aufschmelzen und das glasartige Erstarren der Polymeren, bei dem sich die Seitengruppen aufgrund ihrer flüssigkristallinen Eigenschaften räumlich einheitlich ausrichten. Es wird ferner nicht angegeben, ob die darin beschriebenen substituierten Organopolysiloxane für die Herstellung von Langmuir-Blodgett-Schichten geeignet sind.

Aufgabe der vorliegenden Erfindung ist es, neue Verbindungen bereitzustellen, welche in einfacher Weise erhältlich sind, nichtlinear optische Eigenschaften aufweisen und sich für die Herstellung von neuen Polymeren mit nichtlinear optischen Eigenschaften, insbesondere von neuen Organopolysiloxanen mit Seitengruppen mit nichtlinear optischen Eigenschaften, eignen. Außerdem sollen die neuen Polymeren, insbesondere die neuen Organopolysiloxane für die Herstellung von Langmuir-Blodgett-Schichten geeignet

sein, in denen die Seitengruppen mit nichtlinear optischen Eigenschaften räumlich einheitlich ausgerichtet sind, so daß die neuen Polymere als neue nichtlinear optische Materialien in neuen nichtlinear optischen Anordnungen verwendet werden können.

Überraschenderweise konnte diese Aufgabe durch die eingangs definierten neuen Verbindungen der allgemeinen Formel I mit nichtlinear optischen Eigenschaften gelöst werden. Hierbei war es im Hinblick auf den Stand der Technik überraschend, daß die neuen Verbindungen der allgemeinen Formel I mit nichtlinear optischen Eigenschaften besonders gut für die Herstellung von neuen Polymeren mit nichtlinear optischen Eigenschaften, insbesondere von neuen Organopolysiloxanen mit Seitengruppen mit nichtlinear optischen Eigenschaften, geeignet sind. Darüber hinaus war es überraschend, daß diese neuen Organopolysiloxane sehr leicht Langmuir-Blodgett-Schichten bilden.

Demnach handelt es sich bei dem Gegenstand der vorliegenden Erfindung um die eingangs definierten neuen Verbindungen der allgemeinen Formel I mit nichtlinear optischen Eigenschaften, welche in folgenden der Kürze halber als "erfindungsgemäße Verbindungen I" bezeichnet werden.

Die erfindungsgemäße Verbindungen I werden durch die allgemeine Formel I beschrieben.

In der allgemeinen Formel I bezeichnet die Variable X entweder einen geradkettigen oder verzweigten Alkandiylrest mit 1 bis 20 C-Atomen oder einen geradkettigen oder verzweigten Alkandiylrest mit 2 bis 20 C-Atomen, dessen Kohlenstoffkette durch -O-, -S- und/oder -NR- unterbrochen ist.

Beispiele geeigneter geradkettiger oder verzweigter Alkandiylreste mit 1 bis 20 C-Atomen sind Methylen, Ethylen, Trimethylen, Tetramethylen, Pentamethylen, Hexamethylen, Heptamethylen, Octamethylen, Nonamethylen, Decamethylen, Undecamethylen, Dodecamethylen, Tridecamethylen, Tetradecamethylen, Pentadecamethylen, Hexadecamethylen, Heptadecamethylen, Octadecamethylen, Nonadecamethylen, Eicosamethylen, Ethan-1,1-diyl, Propylen, Butylen-(2,3), 2,3-Dimethyl-butan-1,4-diyl, 1-Methyl-hexan-1,6-diyl, 2,2,4,4-Tetramethyl-hexan-1,6-diyl und 2,2-Dimethyl-propan-1,3-diyl, 2-Oxapropan-1,3-diyl, 2-Thiapropan-1,3-diyl, 3-Oxapentan-1,5-diyl, 3,6-Dioxaoctan-1,8-diyl, 3-Thiapentan-1,5-diyl, 2-Azapropan-1,3-diyl, 2-Methyl-2-azapropan-1,3-diyl, 3-Aza-pentan-1,5-diyl, 3-Aza-pentan-1,4-diyl, 3-Ethyl-3-azapentan-1,5-diyl, 3,6-Diazaoctan-1,8-diyl, 3,6-Dimethyl-3,6-diazaoctan-1,8-diyl und 3-Methyl-3-aza-6-oxa-octan-1,8-diyl, von denen Methylen, Ethylen, Trimethylen, Tetramethylen, Pentamethylen, Hexamethylen und 2-Oxapropan-1,3-diyl gut geeignet sind. Von diesen wird wiederum Methylen ganz besonders bevorzugt verwendet.

In der allgemeinen Formel I bedeutet die Variable R entweder ein Wasserstoffatom, einen geradkettigen oder verzweigten Alkylrest mit 1 bis 6 C-Atomen oder einen Cycloalkylrest.

Beispiele geeigneter geradkettiger oder verzweigter Alkylreste mit 1 bis 6 C-Atomen sind Methyl, Ethyl, Propyl, Isopropyl, n-Butyl, sec.-Butyl, tert.-Butyl, n-Pentyl und n-Hexyl.

Beispiele geeigneter Cycloalkylreste sind Cyclopentyl und Cyclohexyl.

Hiervon wird erfindungsgemäß der Methylrest besonders bevorzugt verwendet.

In der allgemeinen Formel I bedeutet die Variable Y einen nicht-zentrosymmetrischen, ein leicht polarisierbares konjugiertes $\pi$-Elektronensystem sowie mindestens eine endständige Elektronenakzeptorgruppe enthaltender Rest.

Beispiele geeigneter, nicht-zentrosymmetrischer, ein leicht polarisierbares konjugiertes $\pi$-Elektronensystem sowie mindestens eine endständige Elektronenakzeptorgruppen enthaltender Reste Y sind:

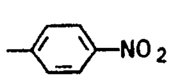 , 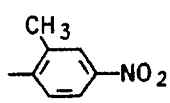 , 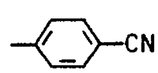 , 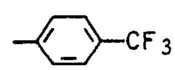 ,

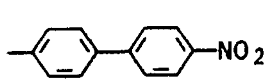 , 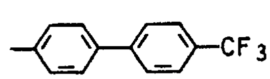 , 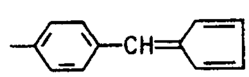 ,

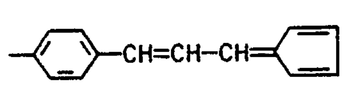 , 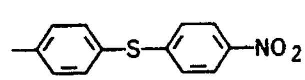 ,

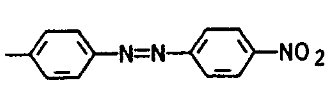 , 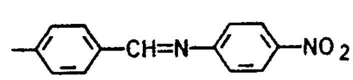 ,

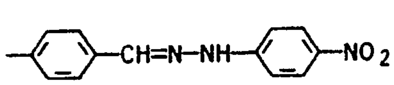 , 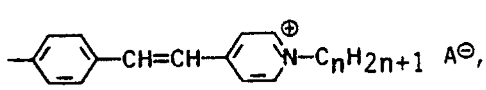 ,

worin
n eine ganze Zahl von 1 bis 22 und $A^{\ominus}$ ein übliches Säureanion wie $Cl^{\ominus}$, $Br^{\ominus}$ oder $HSO_4^{\ominus}$ bezeichnet;

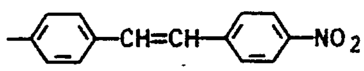 ,

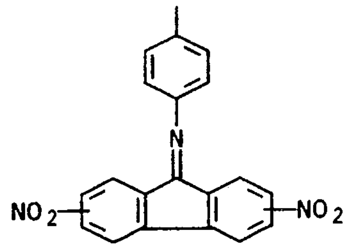 ,

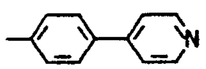 , 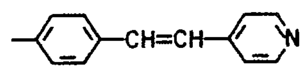 oder 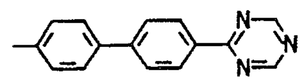 .

Beispiele gut geeigneter Reste Y sind diejenigen, welche Nitro-, Cyano-, Trifluormethyl- oder Fulven-6-yl-Gruppen als endständige Elektronenakzeptorgruppe enthalten, denn die erfindungsgemäßen Verbindungen I mit einem dieser gut geeigneten Reste Y weisen besonders hervorragende anwendungstechnische Eigenschaften auf.

Beispiele vorteilhafter erfindungsgemäßer Verbindungen I sind demnach die erfindungsgemäßen Verbindungen I-1 bis I-8,

I-1 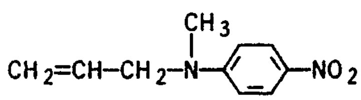 ,

I-2 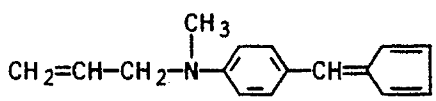 ,

I-3 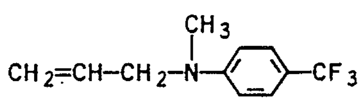 ,

I-4 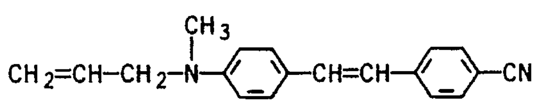 ,

I-5 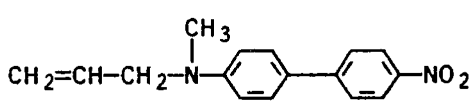 ,

I-6 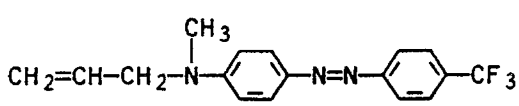 ,

I-7 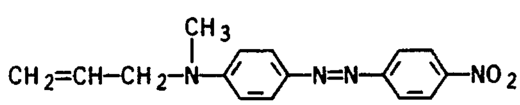 und

I-8 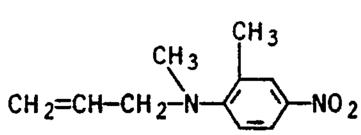 ,

von denen die erfindungsgemäßen Verbindungen I-7 und I-8 ganz besonders vorteilhaft sind.

Methodisch weist die Herstellung der erfindungsgemäßen Verbindungen I keine Besonderheiten auf, sondern es werden hierbei Synthesemethoden verwendet, wie sie in der niedermolekularen organischen Chemie üblich und bekannt sind. So kann beispielsweise die besonders vorteilhafte erfindungsgemäße Verbindung I-7 durch Alkylierung von N-Methylanilin mit Allylchlorid in Toluol in der Gegenwart von Triethylamin und durch Umsetzung des hierbei resultierenden N-Methyl-N-allylanilins mit dem in üblicher und bekannter Weise hergestellten Diazoniumsalz des 4-Nitroanilins hergestellt werden.

Die erfindungsgemäße besonders vorteilhafte Verbindung I-8 kann beispielsweise durch Formylierung von 2-Methyl-4-nitroanilin mit 95 %iger Ameisensäure, Alkylierung des hierbei resultierenden N-Formyl-2-methyl-4-nitroanilins mit Allylchlorid unter Phasentransferbedingungen und Reduktion des hierbei erhaltenen N-Allyl-N-formyl-2-methyl-4-nitroanilins hergestellt werden. Andererseits ist es auch möglich, das Zwischenprodukt N-Allyl-N-formyl-2-methyl-4-nitroanilin unter Bildung des N-Allyl-2-methyl-4-nitroanilins zu hydrolysieren, wonach dieses mit Methyliodid unter Erhalt der erfindungsgemäßen Verbindung I-8 alkyliert wird. Zu diesem Zweck kann auch das Zwischenprodukt N-Allyl-2-methyl-4-nitroanilin durch Alkylierung von N-Formyl-2-methyl-4-nitroanilin mit Allylchlorid hergestellt werden. Daneben ist es auch möglich, das Zwischenprodukt N-Formyl-2-methyl-4-nitroanilin mit Borhydrid in Tetrahydrofuran zu dem entsprechenden N-Methyl-Derivat zu reduzieren, um dieses anschließend mit Allylchlorid unter Erhalt der erfindungsgemäßen Verbindung I-8 zu alkylieren.

Die erfindungsgemäßen Verbindungen I weisen zahlreiche besonderen Vorteile auf.

So lassen sie sich leicht aus üblichen und bekannten, z.T. im Handel erhältlichen Vorprodukten herstellen. Sie weisen nichtlinear optische Eigenschaften auf, welche sie für die Anwendung als nichtlinear optische Materialien in nichtlinear optischen Anordnungen, die den eingangs genannten Zwecken dienen, hervorragend geeignet machen. Hierbei enthalten die resultierenden neuen nichtlinear optischen Materialien mindestens eine der erfindungsgemäßen Verbindungen I oder sie bestehen aus mindestens einer solchen Verbindung I. Die neuen nichtlinear optischen Anordungen enthalten wie die bislang bekannten mindestens einen Träger, welcher dem jeweiligen Verwendungszweck der betreffenden nichtlinear optischen Anordnung angepaßt ist. Darüber hinaus enthalten die neuen nichtlinear optischen Anordnungen mindestens eine Schicht, welche mindestens eine der erfindungsgemäßen Verbindungen I enthält oder welche aus mindestens einer solchen Verbindung I besteht. Hierbei erweisen sich sowohl die neuen nichtlinear optischen Materialien als auch die neuen nichtlinear optischen Anordnungen wegen der Verwendung der erfindungsgemäßen Verbindungen I als den herkömmlichen Materialien und Anordnungen überlegen.

Ein weiterer besonderer Vorteil der erfindungsgemäßen Verbindungen I ist, daß sie sich besonders gut für die Herstellung von Polymeren mit nichtlinear optischen Eigenschaften eignen. Hierbei können die erfindungsgemäßen Verbindungen I durch radikalische Additionspolymerisation selbst Polymere bilden oder sie können über geeignete Reaktionen in bereits vor handene Polymere als Seitengruppen Ia mit nichtlinear optischen Eigenschaften,

$$-CH_2-CH_2-X-\overset{R}{N}-Y \qquad (Ia)$$

worin die Variablen X, R und Y die vorstehend im Detail erläuterte Bedeutung haben,
eingeführt werden. Zu diesen Zwecken können beliebige geeignete Polymere verwendet werden.

Erfindungsgemäß von Vorteil ist indes die Verwendung von Organopolysiloxanen, welche an Siliciumatome gebundene Wasserstoffatome enthalten, weil die betreffenden SiH-Gruppierungen sehr leicht an endständig ethylenisch ungesättigte Verbindungen unter Bildung von SiC-Einfachbindungen addieren. Reaktionen dieser Art sind ebenso bekannt wie die hierfür geeigneten Organopolysiloxane und gehen beispielsweise aus den Patentschriften US-A-4 762 912, US-A-4 358 391, US-A-4 388 453 und US-A-4 410 570 hervor.

Die hierbei resultierenden erfindungsgemäßen Organopolysiloxane enthalten entweder Gruppen der allgemeinen Formel II oder sie bestehen aus diesen.

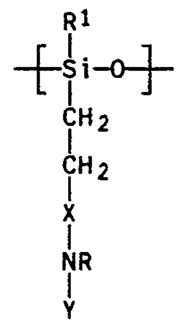

(II)

Hierin haben die Variablen X, R und Y die vorstehend im Detail erläuterte Bedeutung. Dagegen bezeichnet die Variable $R^1$ einen aromatischen Rest wie Phenyl oder sie hat die selbe Bedeutung wie die Variable R.

Die besonderen Vorteile der erfindungsgemäßen Verbindungen I manifestieren sich auch in den vorstehend beschriebenen erfindungsgemäßen Organopolysiloxanen. Auch diese weisen hervorragende nichtlinear optische Eigenschaften auf, so daß auch sie als nichtlinear optische Materialien in nichtlinear optischen Anordnungen der vorstehend genannten Art verwendet werden können.

Insbesondere sind die erfindungsgemäßen Organopolysiloxane für die Herstellung von Langmuir-Blodgett-Mono- und Multischichten geeignet, welche entweder nur aus den erfindungsgemäßen Organopolysiloxanen bestehen oder welche im Falle der Multischichten weitere in "Y-deposition" aufgetragene sonstige Langmuir-Blodgett-Schichten in alternierender Folge enthalten.

Besonders bevorzugt sind die Langmuir-Blodgett-Multischichten, welche Langmuir-Blodgett-Monoschichten aus mindestens einem der erfindungsgemäßen Organopolysiloxane und Langmuir-Blodgett-Monoschichten aus mindestens einem polymeren Amphiphil in "Y-deposition" und in alternierender Folge enthalten.

Ein Beispiel eines besonders gut geeigneten polymeren Amphiphils ist hierbei

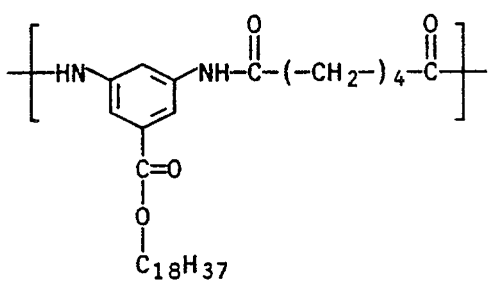

.

Die Herstellung dieser erfindungsgemäßen Langmuir-Blodgett-Mono- und Multischichten bietet methodisch keine Besonderheiten, sondern erfolgt nach der üblichen und bekannten, eingangs beschriebenen Langmuir-Blodgett-Technik.

Hierbei erweisen sich die Seitengruppen in den erfindungsgemäßen Organopolysiloxanen als besonders leicht einheitlich räumlich ausrichtbar, ohne daß hierzu auf schwer herstellbare Seitengruppen mit flüssigkristallinen Eigenschaften oder auf Verfahren zu ihrer zwangsweisen einheitlichen räumlichen Ausrichtung zurückgegriffen werden muß. Im Hinblick auf die Verwendung der erfindungsgemäßen Organopolysiloxane und der erfindungsgemäßen Langmuir-Blodgett-Schichten als nichtlinear optische Materialien ist dies ein ganz besonderer Vorteil.

Sofern es bei oder nach der einheitlichen räumlichen Ausrichtung der Seitengruppen in den erfindungsgemäßen Organopolysiloxanen durch die Langmuir-Blodgett-Technik überhaupt noch notwendig ist, können die erfindungsgemäßen Langmuir-Blodgett-Mono- und -Multischichten unter Zuhilfenahme elektrischer und/oder magnetischer Felder der geeigneten Richtung und des geeigneten Vorzeichens hergestellt und/oder nach ihrer Herstellung, ggf. in diesen Feldern, üblichen und bekannten Verfahren für das Domänenwachstum, wie z.B. der Rekristallisation oder dem Zonenschmelzen, unterzogen werden.

Demgemäß eignen sich die erfindungsgemäßen Organopolysiloxane in hervorragender Weise für die Herstellung neuer nichtlinear optischer Anordnungen, wie sie beispielsweise für die Frequenzverdoppelung, die Frequenzmischung oder für die Lichtwellenleitung verwendet werden oder wie sie in optischen Modulatoren, optischen Multiplexern, optischen logischen Bausteinen oder optischen Verstärkern vorliegen.

Diese neuen nichtlinear optischen Anordnungen enthalten mindestens einen dem entsprechenden Verwendungszweck in Form und Funktion angepaßten Träger, z.B. ein Halbleiterplättchen, und mindestens eine Schicht, insbesondere eine Langmuir-Blodgett-Schicht, welche mindestens ein erfindungsgemäßes Organopolysiloxan enthält oder welche hieraus besteht.

Bei ihrer Verwendung in nichtlinear optischen Anordnungen offenbaren sich weitere besondere Vorteile der erfindungsgemäßen Organopolysiloxane und der erfindungsgemäßen Langmuir-Blodgett-Schichten: So hat ihre exakte zweidimensionale Ausrichtung eine gleichmäßige Dicke und die höchstmögliche Anisotropie der betreffenden Schichten zur Folge. Außerdem sind die betreffenden Schichten stabil gegenüber intensiver Laserstrahlung.

Beispiele

Beispiel 1

Die Herstellung und die physikalischen Eigenschaften der erfindungsgemäßen Verbindung I-8 (N-Allyl-N-Methyl-2-methyl4-nitroanilin);

Versuchsvorschrift:

Für die Herstellung der erfindungsgemäßen Verbindung I-8 wurden zunächst verschiedene Ausgangsprodukte hergestellt, welche dann in unterschiedlicher Art und Weise zu der erfindungsgemäßen Verbindung I-8 umgesetzt wurden.

1.1 Die Herstellung von N-Formyl-2-methyl-4-nitroanilin aus 2-Methyl-4-nitroanilin und Ameisensäure

In einem Kolben mit Rührer und Rückflußkühler wurden 1000 g Ameisensäure vorgelegt. Hierzu gab man 151 g (1 mol) 2-Methyl-4-nitroanilin und erhitzte die resultierende Mischung langsam auf Rückflußtemperaturen. Nach 4 Stunden wurde die Reaktionsmischung auf 5 l Eiswasser gegossen. Der hierbei erhaltene Niederschlag wurde abgesaugt, mit Wasser neutral gewaschen und hiernach bei 60 °C im Vakuum getrocknet. Nach dem Umkristallisieren des so erhaltenen Rohproduktes aus 2,5 l Methanol oder Ethanol erhielt man 171 g N-Formyl-2-methyl-4-nitroanilin (Ausbeute: 95 %) als gelbe Kristalle, welche bei 162 °C schmolzen.

Die gereinigte Verbindung wies noch die weiteren physikalisch chemischen Eigenschaften auf:

- $R_f$ (Aluminiumoxid, Essigester) = 0,6
- $^1$H-NMR-Spektrum (270 MHz, $D_6$-Dimethylsulfoxid DMSO)

$\delta$ (ppm) = 9,85 (breites Singulett $s_{br}$, 1H, NC $\underline{H}$ O)
8,50 ($s_{br}$, 1H, Ar-$\underline{H}$ )
8,25 ($s_{br}$, 1H, N-$\underline{H}$)
8,05 - 8,00 (Multiplett m, 2H, Ar-$\underline{H}$ )
2,38 (Singulett s, 3H, C $\underline{H}_3$)

- $^{13}$C-NMR-Spektrum (67,9 MHz, $D_6$-DMSO)

$\delta$ (ppm) = 160,7, 143,1 142,4, 129,1, 125,5, 122,2, 120,8, 17,8

- IR-Spektrum (KBr)

3380, 1710, 1585, 1529, 1505, 1460, 1339, 1325, 1274, 1260, 1144, 1120, 910, 855, 810, 755, 680 $cm^{-1}$

| • Elementaranalyse ($C_8H_8N_2O_3$: 180,2) | | | |
|---|---|---|---|
| Ber.: | C 53,3 | H 4,5 | N 15,6 |
| Gef.: | C 52,9 | H 4,5 | N 15,5 |

1.2 Die Herstellung von N-Formyl-2-methyl-4-nitroanilin aus 2-Methyl-4-nitroanilin und Ameisensäure/Essigsäureanhydrid

25 g (0,26 mol) Essigsäureanhydrid wurden unter Stickstoff auf 0 °C gekühlt. Bei dieser Temperatur wurden 15 g (0,32 mol) Ameisensäure hinzugetropft. Hiernach ließ man das resultierende Gemisch auftauen, wonach man es während 2 Stunden auf 50 bis 60 °C erwärmte. Nach dem Abkühlen auf Raumtemperatur wurden dem Gemisch 20 ml getrocknetes Tetrahydrofuran hinzugefügt, wonach das so erhaltene Gemisch wieder auf 0 °C abgekühlt wurde. Zu diesem Gemisch wurde eine Suspension von 15,2 g (0,1 mol) 2-Methyl-4-nitroanilin in 100 ml THF hinzugetropft. Die so erhaltene Reaktionsmischung wurde noch für eine Stunde bei 0 °C belassen und hiernach auf Raumtemperatur gebracht. Der hierbei resultierende gelbe Niederschlag wurde abgesaugt, mit Wasser gewaschen und bei 50 °C im Vakuum getrocknet. Hierbei wurden 13,9 g N-Formyl-2-methyl-4-nitroanilin (Ausbeute: 77 %) erhalten. Das Produkt wies dieselben physikalisch chemischen Eigenschaften auf, wie das in der vorstehend beschriebenen Weise (vergleiche Ziffer 1.1) hergestellte.

1.3 Die Herstellung von N-Allyl-N-formyl-2-methyl-4-nitroanilin aus N-Formyl-2-methyl-4-nitroanilin

155 g Kaliumcarbonat wurden in 500 ml Dimethylformamid vorgelegt. Bei der Zugabe von 50 g (0,28 mol) N-Formyl-2-methyl-4-nitroanilin zu diesem Gemisch wurde eine organgefarbene Lösung erhalten, welche sich nach Zugabe von 128,6 g (136,8 ml, 1,68 mol) Allylchlorid blutrot verfärbte. Die so erhaltene Reaktionsmischung wurde über Nacht gerührt und anschließend auf 1,5 l Eiswasser gegossen. Das sich hierbei abscheidende Rohprodukt wurde zweimal mit je 400 ml Dichlormethan aus der wäßrigen Phase extrahiert. Die beiden vereinigten Dichlormethanphasen wurden dreimal mit je 500 ml Wasser gewaschen, anschließend über Natriumsulfat getrocknet und hiernach eingeengt. Es resultierten 58,3 g N-Allyl-N-formyl-2-methyl-4-nitroanilin (Ausbeute: 95 %) als orangefarbenes Öl, welches im Kühlschrank auskristallisierte. Das Produkt wies einen Schmelzpunkt von 56 °C und die weiteren folgenden physikalisch chemischen Eigenschaften auf:

- $R_f$ (Aluminiumoxid, n-Hexan/Essigester, 3/1) = 0,3
- $^1$H-NMR-Spektrum (270 MHz, $D_6$-DMSO)

δ (ppm) = 8,45 (s, 1H, NC H O)
8,25 - 8,05 (m, 2H, Ar-H )
7,55 -7,50 (zwei Doubletts 2d, 1H, Ar-H )
6,00 - 5,75 (m, 1H, $NCH_2C$ H $CH_2$)
5,25 - 5,10 (m, 2H, $NCH_2CHC$ $H_2$)
4,40 - 4,35 (m, 2H, NC $H_2CHCH_2$)
2,4, 2,3 (zwei Singuletts, 2s, 3H, Ar-C $H_3$)

- $^{13}$C-NMR-Spektrum (67,9 MHz, $D_6$-DMSO)

δ (ppm) = 162,2, 162,1, 146,7, 145,4, 144,7, 138,5, 137,7, 133,8, 132,6, 129,7, 129,2, 126,2, 125,5, 122,1, 121,8, 119,7, 118,8, 52,3, 47,7, 18,1

- IR-Spektrum (KBr)

1673, 1518, 1487, 1420, 1390, 1343, 1207, 954, 807, 738 $cm^{-1}$

| • Elementaranalyse ($C_{11}H_{12}N_2O_3$: 220,2) | | | |
|---|---|---|---|
| Ber.: | C 60,0 | H 5,5 | N 12,7 |
| Gef.: | C 59,9 | H 5,7 | N 12,2 |

1.4 Die Herstellung von N-Allyl-2-methyl-4-nitroanilin aus N-Allyl-N-formyl-2-methyl-4-nitroanilin

72 g (0,33 mol) N-Allyl-N-formyl-2-methyl-4-nitroanilin wurden in 500 ml Dimethylformamid gelöst. Die hierbei erhaltene Lösung wurde auf 70 °C erhitzt und mit 300 g 1N Natronlauge versetzt. Die hierbei erhaltene Reaktionsmischung wurde während 4 Stunden bei 70 °C belassen und anschließend auf 3 l Eiswasser gegossen. Der hierbei ausgefällte Niederschlag wurde abgesaugt und nach dem Waschen mit

Wasser im Vakuum bei 50 °C getrocknet. In dieser Weise wurden 49,3 g Rohprodukt (Ausbeute: 85,5 %) erhalten. Das gelbe Rohprodukt wurde bei 50 °C in Ethanol gelöst. Beim Abkühlen der Lösung auf 5 °C kristallisierte N-Allyl-2-methyl-4-nitroanilin in Form gelber Nadeln aus, welche bei 58,5 bis 59,5 °C schmolzen.

Das gereinigte Produkt wies noch die folgenden weiteren physikalisch chemischen Eigenschaften auf:

- $R_f$ (Aluminiumoxid, n-Hexan/Essigester, 3/1) = 0,5
- $^1$H-NMR-Spektrum (270 MHz, $D_6$-DMSO)

$\delta$ (ppm) = 7,95 - 7,90 (m, 2H, Ar-H )
6,55 (d, 1H, Ar-H )
6,50 (s, 1H, N-H )
6,00 = 5,85 (m, 1H, $NCH_2$C H $CH_2$)
5,25 - 5,15 (m, 2H, $NCH_2$C H $CH_2$)
4,00 - 3,90 (m, 2H, NC H $_2CHCH_2$)
2,25 (s, 3H, Ar-C H $_3$)

- $^{13}$C-NMR-Spektrum (67,9 MHz, $D_6$-DMSO)

$\delta$ (ppm) = 152,5, 136,2, 134,9, 125,6, 124,2, 121,8, 115,8, 108,2 45,2, 17,5

- IR-Spektrum (KBr)

3400, 1604, 1588, 1526, 1488, 1463, 1312, 1277, 1240, 1187, 1105, 960, 750 $cm^{-1}$

| • Elementaranalyse ($C_{10}H_{12}N_2O_2$: 192,2) | | | |
|---|---|---|---|
| Ber.: | C 62,5 | H 6,3 | N 14,6 |
| Gef.: | C 62,4 | H 6,4 | N 14,7 |

Das Produkt war hervorragend für die Herstellung der erfindungsgemäßen Verbindung I-8 geeignet.

1.5 Die Herstellung von N-Allyl-2-methyl-4-nitroanilin aus N-Formyl-2-methyl-4-nitroanilin in einem Eintopfverfahren

60 g (0,33 mol) N-Formyl-2-methyl-4-nitroanilin wurden in 500 ml Dimethylformamid gelöst. Die resultierende Lösung wurde nach Zugabe von 184 g (1,33 mol) Kaliumcarbonat auf 50 °C erwärmt. Zu der 50 °C warmen Mischung wurden innerhalb von 50 Minuten 152,8 (2 mol) Allylchlorid hinzugetropft. Nach Beendigung der Alkylierungsreaktion wurde die erhaltene Reaktionsmischung auf 70 °C erwärmt, wonach man 350 g 1N Natronlauge langsam hinzutropfte. Das so erhaltene Gemisch wurde während 2,5 Stunden bei 70 °C gerührt und anschließend auf 3 l Eiswasser gegossen. Der hierbei ausgefällte Niederschlag wurde abgesaugt, mit Wasser neutral gewaschen und anschließend bei 50 °C im Vakuum getrocknet. Es wurden 61 g N-Allyl-2-methyl-4-nitroanilin (Ausbeute: 95 %) erhalten, welches wie unter Ziffer 1.4 beschrieben umkristallisiert wurde und hiernach dieselben physikalisch chemischen Eigenschaften aufwies wie das gemäß Ziffer 1.4 hergestellte.

Das so erhaltene Produkt war gleichfalls hervorragend für die Herstellung der erfindungsgemäßen Verbindung I-8 geeignet.

1.6 Die Herstellung der erfindungsgemäßen Verbindungen I-8 aus N-Allyl-2-methyl-4-nitroanilin

20 g (104 mmol) N-Allyl-2-methyl-4-nitroanilin wurden in 600 ml Dimethylsulfoxid gelöst. Zu der resultierenden Lösung wurden 41,6 g (1,04 mol) gepulvertes Natriumhydroxid hinzugegeben, worauf sich die Lösung tiefrot verfärbte. Zu der tiefroten Lösung gab man 44,3 g (19,6 ml, 312 mmol) Methyliodid auf einmal hinzu, wodurch sich die Lösung rotbraun verfärbte. Die rotbraune Lösung wurde während 1 bis 2 Stunden bei Raumtemperatur gerührt. Hiernach wurde sie auf 3 l Eiswasser gegossen, wonach man das resultierende Gemisch dreimal mit je 500 ml Dichlormethan extrahierte. Die vereinigten Dichlormethanphasen wurden anschließend dreimal mit je 500 ml Wasser gewaschen, über Natriumsulfat getrocknet, filtriert und im Vakuum eingeengt. Hierbei wurde ein braunes Öl (21,4 g, entsprechend einer Ausbeute von 99 %) erhalten, welches durch Chromatographie an Kieselgel (Laufmittel: n-Hexan/Essigester 15/1) gereinigt wurde. Hiernach wurde N-Allyl-N-methyl-2-methyl-4-nitroanilin (erfindungsgemäße Verbindung I-8) in einer

Ausbeute von 19,5 g, entsprechend 90 %, erhalten.

Die erfindungsgemße Verbindung I-8 wies die folgenden physikalisch chemischen Eigenschaften auf:

- $Kp_{0,2}$ ~ 140-145 °C
- $R_f$ (Aluminiumoxid, n-Hexan/Essigester, 15/1): 0,68
(Kieselgel, n-Hexan/Essigester, 15/1): 0,55
- $^1$H-NMR-Spektrum (270 MHz, $D_6$-DMSO)
δ (ppm) = 8,00 - 7,85 (m, 2H, Ar-H )
7,05 - 7,00 (d, 1H, Ar-H )
5,95 - 5,80 (m, 1H, N-$CH_2$C H $CH_2$)
5,35 - 5,15 (m, 2H, N-$CH_2$CHC H $_2$)
3,70 - 3,65 (d, 2H, N-C H $_2$CH$CH_2$)
2,80 (s, 3H, N-C H $_3$)
2,35 (s, 3H, Ar-C H $_3$)
- $^{13}$C-NMR-Spektrum (67,9 MHz, $D_6$-DMSO)
δ (ppm) = 157,8, 140,7, 134,5, 130,3, 126,7, 122,4, 118,2, 117,5 57,5, 39,7, 19,5
- IR-Spektrum (Film)
1603, 1583, 1503, 1449, 1333, 1274, 1233, 1185, 1093, 933 $cm^{-1}$

| • Elementaranalyse ($C_{11}H_{14}N_2O_2$: 206,2) | | | |
|---|---|---|---|
| Ber.: | C 64,1 | H 6,8 | N 13,6 |
| Gef.: | C 64,0 | H 7,0 | N 13,7 |

Die erfindungsgemäße Verbindung I-8 war hervorragend als nichtlinear optisches Material geeignet. Insbesondere eignete sie sich sehr gut für die Herstellung der erfindungsgemäßen Organopolysiloxane mit Seitengruppen mit nichtlinear optischen Eigenschaften. Die erfindungsgemäßen Organopolysiloxane wiederum konnten sehr leicht in der Form von Langmuir-Blodgett-Multischichten auf geeignete Träger aufgebracht werden, wodurch nichtlinear optische Anordnungen mit sehr guten anwendungstechnischen Eigenschaften resultierten.

1.7 Die Herstellung von N-Methyl-2-methyl-4-nitroanilin aus N-Formyl-2-methyl-4-nitroanilin

18 g (0,1 mol) N-Formyl-2-methyl-4-nitroanilin wurden in 500 ml getrocknetem Tetrahydrofuran vorgelegt. Hierzu wurden bei 0 °C 200 ml einer 1-molaren Lösung des Boran-Tetrahydrofuran-Komplexes in Tetrahydrofuran innerhalb von 45 Minuten zugetropft. Hiernach wurde die resultierende Reaktionsmischung auf Raumtemperatur gebracht und danach während 1 Stunde am Rückfluß erhitzt. Im Anschluß daran wurde sie wieder auf 0 °C abgekühlt und mit 130 ml Wasser hydrolysiert. Nach dem Eindampfen des Tetrahydrofurans wurde der verbleibende Rückstand in 300 ml 6 N Salzsäure aufgenommen und darin während 2,5 Stunden am Rückfluß erhitzt. Hiernach wurde die Salzsäurelösung abgekühlt und auf 1 l Eiswasser gegossen. Der hierbei erhaltene gelbe Niederschlag wurde abgesaugt, mit Wasser gewaschen, im Vakuum bei 50 °C getrocknet und aus Ethanol umkristallisiert. Es wurden 15,8 g des Produktes erhalten (Ausbeute: 95 %).

Das umkristallisierte N-Methyl-2-methyl-4-nitroanilin wies die folgenden physikalisch chemischen Eigenschaften auf:

- Schmelzpunkt: 139 °C
- $R_f$ (Aluminiumoxid, Essigester) = 0,75
- $^1$H-NMR-Spektrum (270 MHz, $D_6$-DMSO)
δ (ppm) = 8,00 (dd, 1H, Ar-H )
7,90 (s, 1H, Ar-H )
6,50 (d, 1H, Ar-H )
5,90 ($s_{br}$, 1H, N H )
2,90 (d, 3H, NC H $_3$)
2,15 (s, 3H, Ar-C H $_3$)
- $^{13}$C-NMR-Spektrum (67,9 MHz, $D_6$-DMSO)
δ (ppm) = 153,5, 135,8, 125,2, 124,5, 121,5, 107,2, 29,8, 17,3

• IR-Spektrum (KBr)
3387, 1611, 1589, 1544, 1490, 1325, 1263, 1165, 1096, 750 $cm^{-1}$

| • Elementaranalyse ($C_8H_{10}N_2O_2$: 166,2) | | | |
|---|---|---|---|
| Ber.: | C 57,8 | H 6,1 | N 16,9 |
| Gef.: | C 57,8 | H 6,2 | N 16,7 |

Das Produkt war ebenfalls hervorragend für die Herstellung der erfindungsgemäßen Verbindung I-8 geeignet.

1.8 Die Herstellung der erfindungsgemäßen Verbindung I-8 aus N-Methyl-2-methyl-4-nitroanilin

N-Methyl-2-methyl-4-nitroanilin wurde unter den in Ziffer 1.6 angegebenen Bedingungen alkyliert, nur daß anstelle des Methyliodids die dreifache molare Menge an Allylchlorid verwendet wurde. Die hierbei erhaltene erfindungsgemäße Verbindung I-8 wurde wie unter Ziffer 1.6 angegeben isoliert und gereinigt und wies hiernach auch die dort angegebenen physikalisch chemischen Eigenschaften und besonderen Vorteile und Verwendungsmöglichkeiten auf.

Beispiel 2

Die Herstellung und die Eigenschaften der erfindungsgemäßen Verbindung I-7

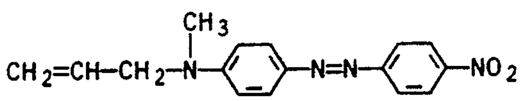 ;

Versuchsvorschrift:

2.1 Die Herstellung von N-Allyl-N-methylanilin

Für die Herstellung der erfindungsgemäßen Verbindung I-7 wurde zunächst N-Allyl-N-methylanilin hergestellt. Hierzu wurden 214 g (2 mol) N-Methylanilin, 202 g (2 mol) Triethylamin in einem Liter Toluol gelöst, wonach die resultierende Lösung auf 80 bis 85 °C erwärmt wurde. Zu dieser Lösung tropfte man unter Rühren 153 g (2 mol) Allylchlorid hinzu, wonach das resultierende Reaktionsgemisch während 1,5 Stunden bei diesen Temperaturen gerührt wurde. Nach der Beendigung der Reaktion wurde das ausgefallene Triethylammoniumchlorid abfiltriert und mit 100 ml Toluol gewaschen. Die beiden toluolischen Lösungen wurden vereinigt und im Vakuum eingeengt. Der hierbei verbleibende Rückstand enthielt 34 % unumgesetztes N-Methylanilin und 66 % N-Allyl-N-methylanilin, welches durch Vakuumdestilliation (3 mbar, 75 bis 76 °C Übergangstemperatur) in einer Reinheit von 99,3 % erhalten werden konnte. Das Produkt wies das folgende $^1$H-Kernresonanzspektrum auf:
$\delta$ (ppm) = 7,25 - 7,15 (m, 2H, Ar-H )
6,75 - 6,70 (m, 3H, Ar-H )
5,90 - 5,75 (m, 1H, N-$CH_2$C H = $CH_2$)
5,20 - 5,10 (m, 2H, N-$CH_2$-CH = C $H_2$)
3,90 (d, 2H, N-C $H_2$-CH = $CH_2$)

2.2 Die Diazotierung von 4-Nitroanilin

Für die Herstellung der erfindungsgemäßen Verbindung I-7 wurde des weiteren 4-Nitroanilin diazotiert. Hierzu wurden 27,6 g (0,2 mol) 4-Nitroanilin in 100 ml konz. Salzsäure und 20 ml Wasser aufgekocht und hiernach in 200 ml kaltes Wasser eingerührt. Man kühlte die resultierende Lösung auf -10 °C und gab 48 ml einer 30 %igen wäßrigen Natriumnitritlösung tropfenweise hinzu, so daß die Temperatur bei -10 °C gehalten wurde. Das resultierende Reaktionsgemisch wurde 30 Minuten lang gerührt, wonach man das hiernach verbleibende überschüssige Natriumnitrit mit Harnstoff zerstörte, was mit Hilfe von Kaliumiodidpapier überwacht wurde. Hierbei wurde darauf geachtet, daß die Temperatur der Reaktionsmischung 0 °C nicht überstieg.

2.3 Die Herstellung der erfindungsgemäßen Verbindung I-7 durch Azokupplung

29,4 g (0,2 mol) N-Allyl-N-methylanilin wurden in 30 ml konz. Salzsäure und 150 ml Wasser aufgelöst. Diese Lösung wurde bei -10 °C zum Diazoniumsalze des 4-Nitroanilins hinzugegeben. Hiernach wurde mit ca. 100 g Kaliumacetat ein pH-Wert von 4 eingestellt, wodurch der Azofarbstoff (die erfindungsgemäße Verbindung I-7) als voluminöser roter Niederschlag ausgefällt wurde. Nach 2,5 Stunden Standzeit wurde der rote Azofarbstoff abgesaugt, mit Wasser neutral gewaschen und getrocknet. Das erhaltene Rohprodukt wurde aus Ethanol umkristallisiert und fiel dabei in Form von dunkelgrün-metallic schimmernden Nadeln an. Es wurden 42 g der erfindungsgemäßen Verbindung I-7 erhalten, was einer Ausbeute von 71 % entsprach.

Das Produkt wies die folgenden physikalisch chemischen Eigenschaften auf:

Schmelzpunkt: 120 bis 120,5 °C

- $R_f$ ($SiO_2$, n-Hexan/Essigester, 3:1) = 0,58
- $^1H$-NMR-Spektrum (250 MHz, $CDCl_3$)

δ (ppm) = 8,25/7,90 (Kopplungsmuster zweier Kerne ähnlicher chemischer Verschiebung des Systems AB, 4H, Ar-H )
7,90/6,65 (AB, 4H, Ar-H )
5,95 - 5,75 (m, 1H, N-$CH_2$-C H = $CH_2$)
5,25 - 5,10 (m, 2H, N-$CH_2$-CH = C $H_2$)
4,10 (d, 2H, N-C $H_2$-CH = $CH_2$)
3,10 (s, 3H, N-C $H_3$)

- $^{13}C$-NMR-Spektrum (67,9 MHz, $CDCl_3$)

δ (ppm) = 157,0, 152,9, 147,7, 144,2, 132,2, 126,1, 126,1, 124,7, 124,7, 122,7, 122,7, 116,9, 111,8, 111,8, 54,9, 38,3

- IR-Spektrum (KBr)

1601, 1586, 1519, 1508, 1379, 1333, 1308, 1135, 1105, 1097 $cm^{-1}$

Die erfindungsgemäße Verbindung I-8 wies ausgezeichnete nichtlinear optische Eigenschaften auf und war deshalb hervorragend als nichtlinear optisches Material für die Herstellung von nichtlinear optischen Anordnungen geeignet. Insbesondere eignete sie sich hervorragend für die Herstellung von Organopolysiloxanen mit Seitengruppen mit nichtlinear optischen Eigenschaften. Diese erfindungsgemäßen Organopolysiloxane konnten in einfacher Weise zu Langmuir-Blodgett-Multischichten geformt werden, welche aufgrund ihrer hervorragenden anwendungstechnischen Eigenschaften in neuen nichtlinear optischen Anordnungen verwendet werden konnten.

## Ansprüche

1. Verbindungen der allgemeinen Formel I mit nichtlinear optischen Eigenschaften,

$$CH_2=CH-X-\overset{R}{N}-Y \qquad (I)$$

worin die Variablen die folgende Bedeutung haben:

X geradkettiger oder verzweigter Alkandiylrest mit 1 bis 20 C-Atomen oder

X geradkettiger oder verzweigter Alkandiylrest mit 2 bis 20 C-Atomen, dessen Kohlenstoffkette durch -O-, -S- und/oder -NR- unterbrochen ist;

R Wasserstoffatom, geradkettiger oder verzweigter Alkylrest mit 1 bis 6 C-Atomen oder Cycloalkylrest,

Y nicht-zentrosymmetrischer, ein leicht polarisierbares konjugiertes $\pi$-Elektronensystem sowie mindestens eine endständige Elektronenakzeptorgruppe enthaltender Rest.

2. Die Verbindungen I nach Anspruch 1, dadurch gekennzeichnet, daß die Variablen die folgende Bedeutung haben:

X Methylen, Ethylen, Trimethylen, Tetramethylen, Pentamethylen, Hexamethylen oder 2-Oxapropan-1,3-diyl;

R Methylrest.

3. Verwendung der Verbindungen I gemäß Anspruch 1 oder 2 für die Herstellung von Polymeren mit nichtlinear optischen Eigenschaften.

4. Polymere mit nichtlinear optischen Eigenschaften, welche Seitengruppen der allgemeinen Formel Ia enthalten,

$$-CH_2-CH_2-X-\overset{R}{N}-Y \qquad (Ia)$$

worin die Variablen die folgende Bedeutung haben:

X geradkettiger oder verzweigter Alkandiylrest mit 1 bis 20 C-Atomen oder

X geradkettiger oder verzweigter Alkandiylrest mit 2 bis 20 C-Atomen, dessen Kohlenstoffkette durch -O-, -S- und/oder -NR- unterbrochen ist;

R Wasserstoffatom, geradkettiger oder verzweigter Alkylrest mit 1 bis 6 C-Atomen oder Cycloalkylrest;

Y nicht-zentrosymmetrischer, ein leicht polarisierbares konjugiertes $\pi$-Elektronensystem sowie mindestens eine endständige Elektronenakzeptorgruppe enthaltender Rest.

5. Die Polymeren nach Anspruch 4, dadurch gekennzeichnet, daß die Variablen die folgende Bedeutung haben

X Methylen, Ethylen, Trimethylen, Tetramethylen, Pentamethylen, Hexamethylen oder 2-Oxapropan-1,3-diyl;

R Methylrest.

6. Die Polymeren nach Anspruch 4 oder 5, dadurch gekennzeichnet, daß es sich hierbei um Organopolysiloxane handelt.

7. Verwendung der Verbindungen I gemäß Anspruch 1 oder 2 und der Polymeren mit Seitengruppen Ia gemäß einem der Ansprüche 4 bis 6 als nichtlinear optische Materialien.

8. Nichtlinear optische Materialien, welche die Verbindungen I gemäß Anspruch 1 oder 2 und/oder die Polymeren mit Seitengruppen Ia gemäß einem der Ansprüche 4 bis 6 enthalten oder welche hieraus bestehen.

9. Nichtlinear optische Anordnung, welche mindestens einen Träger und mindestens eine Schicht aufweist, die die Verbindungen I gemäß Anspruch 1 oder 2 und/oder die Polymeren mit Seitengruppen Ia gemäß einem der Ansprüche 4 bis 6 enthält oder die hieraus besteht.

10. Verwendung der Polymeren gemäß Anspruch 6 für die Herstellung von Langmuir-Blodgett-Schichten.

11. Langmuir-Blodgett-Schichten, welche mindestens ein Polymeres gemäß Anspruch 6 enthalten oder welche hieraus bestehen.

12. Nichtlinear optischte Anordnung, welche mindestens einen Träger und mindestens eine Langmuir-Blodgett-Schicht aufweist, die mindestens ein Polymeres gemäß Anspruch 6 enthält oder die hieraus besteht.

Europäisches Patentamt

# EUROPÄISCHER RECHERCHENBERICHT

Nummer der Anmeldung

EP 90116216.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | GB - A - 2 209 169 (THE PLESSEY COMP.) * Ansprüche 1,7-13; Seite 10, Zeilen 1-19; Zusammenfassung -- | 1-12 | C 07 C 211/52<br>C 07 C 245/08<br>G 02 F 1/35<br>C 08 G 77/388 |
| X | CHEMCIAL ABSTRACTS, Band 83, Nr. 8, 25. August 1975, Columbus, Ohio, USA A.I. KOSTYUKOV et al. "Synthesis of allyl derivatives of azo dyes and their capacity for copolymerization with vinyl monomers" Seite 173, Spalte 1, Zusammenfassung-Nr. 61 596j * Izv. Vyssh. Ucheb. Zaved., Khim. Khim. Tekhnol. 1974, 17(3), 419-22 * -- | 1,2 | |
| P,X | US - A - 4 913 836 (EAST) * Ansprüche 1,2,4,5,8-11 * ---- | 4,6-8 | RECHERCHIERTE SACHGEBIETE (Int. Cl.5)<br>C 07 C 211/00<br>C 07 C 255/00<br>C 07 C 245/00<br>C 08 G 77/00<br>G 02 F<br>C 09 K |

Der vorliegende Recherchenbericht wurde für alle Patentansprüche erstellt.

| Recherchenort | Abschlußdatum der Recherche | Prüfer |
|---|---|---|
| WIEN | 03-12-1990 | KÖRBER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument